# EUROPEAN PATENT APPLICATION

(11) **EP 1 002 543 A1**
(43) Date of publication of application: **24.05.2000**
(21) Application number: 99121973.4
(22) Date of filing: 10.11.1999
(51) Int. Cl.: A61K 38/36, G01N 33/68, C07K 14/75, C07K 14/78

(54) **Pharmaceutical compositions for the therapy of glioma or prostate cell proliferative disorders containing cyclic rgd compounds**

(30) Priority: 12.11.1998 US 108387
(71) Applicant: JCR PHARMACEUTICALS CO., LTD., Ashiya, Hyogo 659-0021 (JP)
(72) Inventor: Subhendra, Chattopadhyay, San Diego, CA 92130 (US)
(74) Representative: Reinhard - Skuhra - Weise & Partner

(57) **Abstract**

Provided is use of a cyclic RGD sequences for the manufacture of a pharmaceutical composition to treat cell proliferative disorders. Such treatments include the inhibition and amelioration of cell proliferative disorders such as neuronal cell proliferative disorders, including glioblastoma, as well as prostate hyperplasia or neoplasia. Also provided are methods for identifying compounds useful in treating cell proliferative disorders associated with αᵥβ₃, and methods for identifying cell proliferative disorders that are susceptible to treatment with cyclic RGD sequences.

## Description

### TECHNICAL FIELD

The present invention relates generally to the treatment of cell proliferative disorders and more specifically to a pharmaceutical composition for the treatment or inhibition of such disorders as well as to the use of cyclic RDG for the manufacture of such a pharmaceutical composition.

### BACKGROUND INFORMATION

Cell proliferative disorders including tumor cells and cancer are a major cause of mortality and morbidity in the world. Glioma or tumors derived from glial cells of the central nervous system (CNS) are the most frequent malignant brain tumors in adults and are invariably fatal. Malignant gliomas account for approximately one-third of all primary brain tumors in adults, approximately 5,000 new cases in the United States annually. The median survival of patients with glioblastoma is approximately 12 months.

Recently, integrins have been shown to play an important role in angiogenesis, or the growth of new blood vessels. Integrins are heterodimeric transmembrane proteins consisting of α and β subunits, and are responsible for cell adhesion to a variety of extracellular matrices (such as, fibronectin, vitronectin, and collagens), as well as cell-to-cell adhesion. To date, 16 α and 8 β subunits (in 21 different combinations) have been reported. One of these combinations, αᵥβ₃ has been identified as a marker of angiogenic vascular tissue (blood vessel) in humans.

### SUMMARY OF THE INVENTION

The present invention provides a method and a pharmaceutical composition for treating a subject with an αᵥβ₃ receptor-mediated cell proliferative disorder. The subject is treated through administering an effective amount of an RGD sequence thereby altering αᵥβ₃ receptor-mediated binding. One embodiment the RGD sequence is cyclic RGD molecule. The method and pharmaceutical composition are useful in inhibiting any cell proliferative disorder in which the cells express αᵥβ₃.

In one embodiment the invention provides a method of treating a glioma or prostate cell proliferative disorder in a subject by administering to the subject a therapeutically effective amount of an RGD sequence which inhibits αᵥβ₃ receptor-mediated binding of the glioma or prostate cell.

In another embodiment, the invention provides a pharmaceutical composition for the treatment of an αᵥβ₃ receptor-mediated glioma or prostate cell proliferative disorder in the subject, wherein the composition contains as a pharmacologically active principle a cyclic RGD (cRGD) sequence.

In another embodiment, the invention provides a method of inhibiting a glioma or prostate cell proliferative disorder wherein the glioma or prostate cell expresses αᵥ β₃ by contacting the glioma or prostate cell with an RGD sequence.

In a further embodiment, the invention provides a pharmaceutical composition_ for the inhibition of occurrence of an αᵥβ₃ receptor-mediated glioma or prostate cell proliferative disorder in the subject, wherein the composition contains as a pharmacologically active principle a cyclic RGD (cRGD) sequence.

In a further embodiment, the invention provides the use of a cyclic RGD (cRGD) sequence as a pharmacologically active principle for the manufacture of a composition for the treatment of an αᵥβ₃ receptor-mediated glioma or prostate cell proliferative disorder in the subject.

In a further embodiment, the invention provides the use of a cyclic RGD (cRGD) sequence as a pharmacologically active principle for the manufacture of a composition for the inhibition of occurrence of an αᵥβ₃ receptor-mediated glioma or prostate cell proliferative disorder in the subject.

In a further embodiment, the invention provides a method for identifying a compound which modulates αᵥβ₃ receptor-mediated cell proliferative disorder by contacting an αᵥβ₃ with a candidate compound suspected of having cell proliferation modulating activity and detecting binding of the compound to αᵥβ₃.

The invention also provides a method of inhibiting the growth of a glioma or prostate cell expressing αᵥβ₃ by contacting the glioma or prostate cell with an inhibiting effective amount of a peptide comprising an RGD sequence.

In another embodiment, the invention provides a method of inhibiting an αᵥβ₃ receptor-mediated cell proliferative disorder in a subject having or at risk of having such a disorder, comprising contacting the subject with a therapeutically effective amount of a peptide comprising the RGD sequence. In another embodiment, the peptide is provided in combination with at least one other chemotherapeutic agent.

In yet another embodiment, the invention provides a method for identifying a cell proliferative disorder that is susceptible to treatment with an RGD peptide by contacting αᵥβ₃ of a cell with the RGD sequence and measuring the effect on αᵥβ₃ receptor-mediated binding.

In another embodiment, the invention provides methods for identifying an agent which inhibits cell proliferation by contacting a sample containing an αᵥβ₃ receptor with a candidate compound suspected of affecting cellular interactions with αᵥβ₃ and detecting an effect on αᵥβ₃ receptor mediated binding.

The details of one or more embodiments of the invention are set forth in the accompanying drawings and the description below. Other features, objects, and, advantages of the invention will be apparent from the description and drawings, and from the claims.

### DESCRIPTION OF DRAWINGS

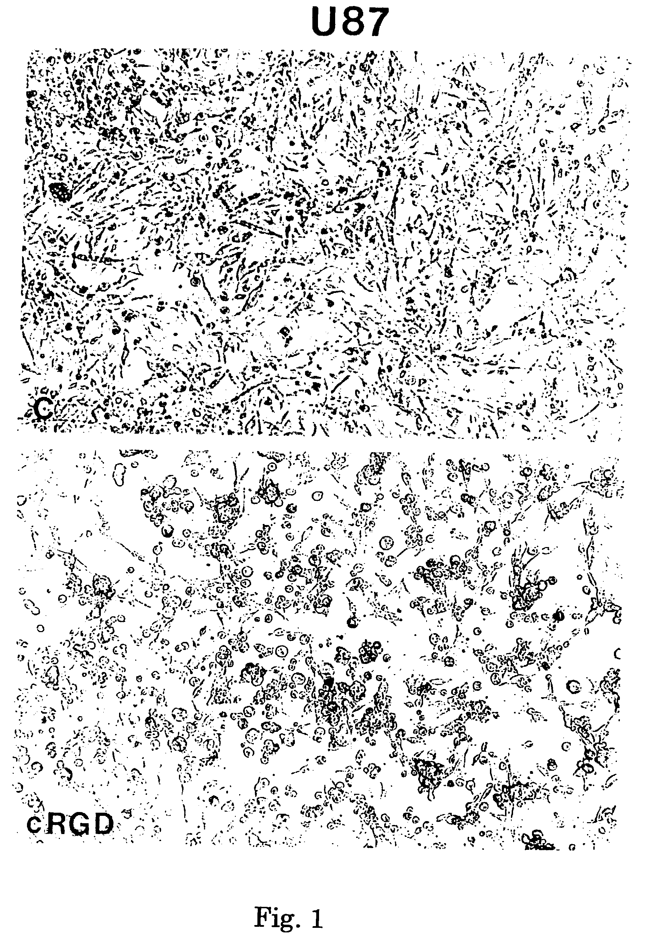

FIG.1 shows cRGD treated U87 cells (cRGD) in culture compared with control U87 cells (C).
FIG.2 shows cRGD treated U251 cells (cRGD) in culture compared with control U251 cells (C).
FIG.3 shows the effect of cRGD on spheroid cultures of U87MG cells (C=control ; cRGD= cyclic RGD treated; and RGD= linear RGD treated).
Fig. 4A and B show the effect of caspase-3 and caspase-9 inhibitors, DEVD-FMK (panel A) and LEHD-FMK (panel B), respectively, on U-87MG cell death induced by cRGDfV.

### DETAILED DESCRIPTION OF THE INVENTION

The present invention relates to a method of detecting, diagnosing, and treating cell proliferative disorders, such as neoplasm or cancer, using cyclic RGD sequences, peptides or compounds containing RGD and functional equivalents thereof. Human gliomas represent a major problem in cancer biology. The present invention indicates that cyclic RGD (cRGD) can induce cell death of human glioma cells by blocking the activity/property of αᵥβ₃ integrin.

The RGD sequences of the invention are useful in that they are capable of binding integrins, such as an αᵥβ₃ integrin or receptor molecule, and therefore can effect αᵥβ₃ receptor-mediated binding. Such integrins are used as attachment mediator for binding of a cell in or on an extracellular matrix material. Such binding is part of the growth and differentiation process of cells. Thus, the RGD sequences of the invention can be useful in inhibiting and altering the growth and differentiation of any cell expressing αᵥβ₃, for example.

In its broadest sense, the present invention allows the treatment or inhibition of any αᵥβ₃ -associated disorder in any organ, tissue, or cell *in vivo* or *ex vivo.* Additionally, the present invention allows for the detection or identification of compounds that can be used to treat a glioma or prostate αᵥβ₃ cell proliferative disorder and cells that can be treated using the methods of the invention.

As used herein, the term "RDG sequence" or "RGD peptide" or "RGD compound" means a molecule having at least one Arg-Gly-Asp sequence that functions to inhibit or block a binding site of an integrin molecule, such as αᵥβ₃. As used herein, the term "cyclic RGD sequence" or "cylcic RGD molecule" means a cyclic sequence or molecule comprising an "RGD sequence" as defined above.

Integrin receptors can bind a variety of RGD sequences of variety lengths (see, for example, Ruoslahti *et al*., In Morphoregulatory Molecules, G.M.Edelman *et al*., eds.(1990); Ruoslahti, J. Chin. Invest. 87:1-5 (1991)). Thus, it is intended that the length of an RGD peptide can vary, for example, from four amino acids up to 100 amino acids or more. More likely, RGD peptide will be from about 5 to about 50 amino acids, more likely from about six to about 25 amino acids. Moreover, it is recognized that the amino acids or other entities that flank the RGD sequence can vary without destroying activity of the molecule. As such, variation of flanking amino acids are specifically contemplated, so long as the variant does not completely lose its activity.

Additionally, it is intended that the RGD sequence includes any compound having an amino acid sequence that is functionary equivalent to the sequence Arg-Gly-Asp. For example, one skilled in the art will recognize that substitution of amino acids can be made using natural amino acids which result in a conservative change, or also also using non-natural or synthetic amino acids that result in a peptide having similar or equivalent functionality. Such amino acids include lysine, homoarginine, N-methyl arginine or a mimic of these amino acids which are functionally equivalent to arginine. Similarly, functionally equivalent amino acid for Gly and Asp can be readily identified by one skilled in the art. An example of a particular equivalent amino acid is where R (Arg) is substituted with K (Lys). Other examples of functional RGD equivalents include amino acid derivatives and mimics, such as those described, for example, in U.S. Patent Nos. 5,612,311 and 5,858,972, which are incorporated herein by reference.

As used herein, "mimic" means an amino acid or analog that has a similar functional characteristic or biological activity. Such characteristics include hydrophilicity, hydrophobicity, side chains and pH. Additionally, the term "amino acid" is meant to include naturally occurring as well as non-naturally occurring amino acids and imino acid, analogs and mimics. One skilled in the art, will recognize that unless indicated otherwise both (L)-amino acids and (D)-amino acids are encompassed by the present definitions and invention. For example, it may be desirable to include one or more (D)-amino acids to increase the stability of the peptide *in vivo*.

In another aspect of the invention the RGD sequence encompasses cyclic molecules of RGD. Such sequence, which are designed to include the sequence Arg-Gly-Asp for binding to αᵥβ₃ integrin, include:
(1) GRGDEPDG (i.e., Gly-Arg-Gly-Asp-Glu-Pro-Asp-Gly: SEQ ID NO:1);
(2) RGDNIE-NH₂ (i.e., Arg-Gly-Asp-Asn-Ile-Glu-NH₂ : SEQ ID NO:2: C-terminus protected as an amide.);
(3) G-Pmc-RGDCA (i.e., Gly-Pmc-Arg-Gly-Asp-Cys-Ala : Pmc= β , β ― penta methylene);
(4) G-Pen-GRGDNYCA (i.e., Gly-Pen-Gly-Arg-Gly-Asp-Asn-Tyr-Cys-Ala : Pen= penicillamine);
(5) G-Pen-GERGDNYCA (i.e., Gly-Pen-Gly-Glu-Arg-Gly-Asp-Asn-Tyr-Cys-Ala);
(6) G-Pen-AARGDVPCANH₂ (i.e., Gly-Pen-Ala-Ala-Arg-Gly-Asp-Val-Pro-Cys-Ala-NH₂);
(7) G-Pen-ELRGDGWCNH₂ (i.e., Gly-Pen-Glu-Leu-Arg-Gly-Asp-Gly-Try-Cys-NH₂);
(8) G-Pen-GFRGDEPCNH₂ (i.e., Gly-Pen-Gly-Phe-Arg-Gly-Asp-Glu-Pro-Cys-NH₂);
(9) K-Pen-GFRGDEPCR (i.e., Lys-Pen-Gly-Phe-Arg-Gly-Asp-Glu-Pro-Cys-Arg);
(10) K-Pen-GFRGDDPCR (i.e., Lys-Pen-Gly-Phe-Arg-Gly-Asp-Asp-Pro-Cys-Arg) ;
(11) Ac-Pen-AARGD-Orn-PC-NH₂ (i.e., Ac-Pen-Ala-Ala-Arg-Gly-Asp-Orn-Pro-Cys-NH₂ : Orn=ornithine);
(12) (Mpa)-R-G-D-(YOMe)-(t-BuG)-C-NH₂ (i.e., (Mpa)-Arg-Gly-Asp-(YOMe)-(t-BuG)-Cys-NH₂ : Mpa=β -mercaptopropionic acid; YOMe=O-methyl-tyrosine; t-BuG=tert-butyl Glicine);
(13) (Mpa)-R-G-D-(Cha)-(t-BuG)-C-NH₂ (i.e., (Mpa)-Arg-Gly-Asp-(Cha)-(t-BuG)-Cys-NH₂ : Cha=cyclohexyl-alanine ); and
(15) the structure depicted in the following formula:

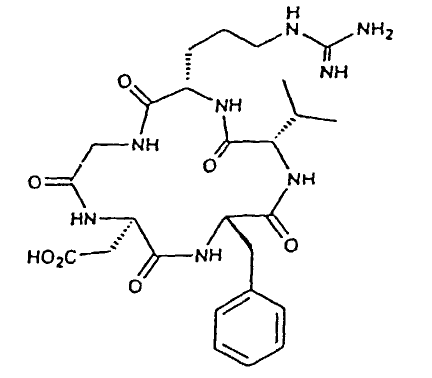

In an additional aspect, the invention also includes a linear RGD and functional equivalents thereof. Additional cyclic or conformationally constrained RGD molecules are described in U.S. Patent Nos. 5,547,936; 5,827,821; 5,672,585; 5,627,263 and 5,912,234, which are incorporated herein by reference. Such cyclic RGD molecules having disulfide linkages or other intramolecular bonds in various positions relative to the RGD motif are included for use herein.

RGD sequences or peptides can be synthesized, for example, by an automated synthesizer. Such automated synthesizers are common in the art and include for example, Applied Biosystems, Inc. Model 431A. For techniques in using an automated synthesizer, see Steward and Yound, In Solid Phases Peptide Synthesis. 2nd ed. Pierce Chemical Co., Rockford, IL., (1984), which is incorporated herein by reference.

As used herein, to "inhibit" or "inhibiting" activity is to reduce that activity a measurable amount, such as a reduction of at least 30% or more. Where there are multiple different activities that may be inhibited (for example, blocking binding of a receptor mediated binding and initiation of secondary messenger activity), the reduction of any single activity (with or without reducing other activities) is sufficient to fall within the scope of this definition.

The present invention provides a method for treating a subject with an αᵥβ₃-associated cell proliferative disorder. For example, many glioma cells reqiure αᵥβ₃ receptor for attachment to the extracellular matrix in order to remain viable, therefore, it is possible to design appropriate therapeutic or diagnostic techniques directed to inhibit or prevent such receptor-mediated binding. Thus, where a glioma cell-proliferative disorder is associated with the expression of αᵥβ₃, sequences that modulate binding of glioma cells to the extracellular matrix can inhibit their growth and metastasis.

The term "cell-proliferative disorder" denotes malignant, non-malignant, as well as benign hyperplastic cell populations which often appear to differ from the surrounding tissue both morphologically and genotypically. Such disorders may be associated, for example, with over-expression of αᵥβ₃. Essentially, any cell disorder which is etiologically linked to expression of αᵥβ₃ integrin could be treated with a molecule which modulates αᵥβ₃ receptor-mediated binding. Such cell proliferative disorders include glioma and prostate cell disorders. Glioma cell proliferative disorder can be any number of disorders including neoplasms, neural cell disorders and tumors.

The term "modulate" or "regulates" envisions the augmentation of cellular binding to a substrate, cell growth, and/or cellular differentiation.

The RGD sequence can effectively inhibit and treat a glioma or glioblastoma cell proliferative disorders. The RGD sequence can be delivered to a glioma cell in culture or to a glioma cell or tissue in a subject *in vivo.* Typically the subject is a mammal and more typically a human. Binding of the RGD sequence to αᵥβ₃ can be used to inhibit cell binding, growth and metastasis associated with cell proliferative disorders. Furthermore, the RGD sequence can be used in combination with other chemotherapies, the combination therapy may prove more effective than either RGD sequence or chemotherapeutic compound alone.

In order for the target glioma cell, tissue, or subject to be rendered susceptible to the RGD sequence in accordance with a method of the invention, the αᵥβ₃ cell must be exposed to the sequence. Typically, the αᵥβ₃ is present on the cell thereby facilitating contact with the RGD sequence. *In vitro* therapy may be accomplished by a number of procedures, including, for example, simple incubation of a cell or tissue with the RGD sequence in a suitable nutrient medium for a period of time suitable to inhibit αᵥβ₃ receptor-mediated binding.

RGD molecules of the invention can be delivered alone or in conjunction with other agents such as chemotherapeutic drugs, ribozymes or antisense molecules. For example, chemotherapeutic drugs include: bleomycin, neocarcinostatin, suramin, doxorubicin, taxol, mitomycin C and cisplatin. Additional chemotherapeutics are known in the art and are contemplated for use in combination with RGD molecules. The RGD molecules of the invention can be delivered before, during, or after a surgical procedure, radiation treatment or chemotherapy to treat a glioma or prostate cell proliferative disorder.

Additionally, the RGD sequence of the present invention may be administered *ex vivo* by harvesting a cell or tissue from a subject, treating the cell or tissue with the sequence, then returning the treated cell or tissue to the subject.

Many of the methods as described herein can be performed *in vivo* or *ex vivo.* The RGD molecule of the invention can administered to provide *in vivo* therapy to a subject having a glioma cell proliferative disorder. Such therapy can be accomplished by administering *ex vivo* or *in vivo* as the case may be, a therapeutically effective amount of an RGD sequence. The term "therapeutically effective" means that the amount of RGD sequence administered is of sufficient quantity to suppress or inhibit, to some beneficial degree, αᵥβ₃ receptor-mediated binding of a αᵥβ₃ containing cell thereby inhibiting cell growth or promoting cell death.

For example, studies to determine *in vivo* efficacy of cRGD ("c" denotes "cyclic") in an intracranial SCID (severe combined immunodeficient)_mouse glioma model can be carried out by implanting, intracranially 1×10⁵ to 1×10⁶ glioma cells in 5 µl saline using a syringe fitted with a 30 G 1/2 inch needle. Different times after the glioma cell injection, saline or saline containing various doses (1, 10 or 100 mg/kg/day) of peptide or RGD sequence will be injected intratumorally with 10 µl of the relevant preparation. Administration of the RGD sequence can be performed intraperitoneally or intravenously. A control peptide (e.g., non RGD peptide) can be administered in a similar fashion. The ability of the RGD sequence to reduce or inhibit cell proliferative disorders will be evaluated by tumor growth and metastasis. Both treated and untreated mice will be followed until death, and mice surviving beyond 90 days will be killed for histological analysis. Survival times can be correlated with histopathology for each group and the data converted to generate Kaplan-Meier plots for each group. Median survival times will be estimated for each group. Statistical significance of tumor dose to survival will be calculated. The studies will demonstrate the efficacy of the cRGD *in vivo.*

RGD molecules according to the present invention can be administered to the subject in any acceptable manner including orally, by injection, using an implant, nasally, inhalation and the like. Oral administration includes administering an RGD sequence of the present invention in tablets, suspension, implants, solutions, emulsions, capsules, powders, syrups water composition, and the like. Nasal and inhalation administration includes administering the composition of the present invention in sprays, aerosol solutions and the like. Injections and implants permit precise control of the number,_timing and dosage levels useful for administration, with injections being preferred.

The RGD molecules_useful in the method of the invention can be administered parenterally by injection or by gradual perfusion over time. Administration may be intracranially,_intravenously, intra-peritoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's intravenous vehicles include fluid and nutrient replenishers, electrolyte replenishers (such as those based on Ringer's dextrose), and the like. Preservatives and other additives may also be present, such as antimicrobials, anti-oxidants, chelating agents and inert gases and the like.

The actual dosage of an RGD sequence, formulation, or composition that modulates a cell proliferative disorder depends on many factors, including the size and health of an individual, however, one of ordinary skill in the art can use the following teachings (Spilker B., *Guide to Clinical Studies and Developing Protocols*, Raven Press Books, Ltd., New York, 1984, pp. 7-13, 54-60; Spilker B., *Guide to Clinical Trials*, Raven Press, Ltd., New York, 1991, pp. 93-101; Craig C., and R. Stitzel, eds., *Modern Pharmacology*, d. ed., Little, Brown and Co., Boston, 1986, pp. 127-33; T. Sleight, ed., *Avery's Drug Treatment: Principles and Practice of Clinical Pharmacology and Therapeutics*, 3d ed., Williams and Wilkins, Baltimore, 1987, pp. 50-56; R. Tallarida, R. Raffa and P. McGonigle, *Principles in General Pharmacology*, Springer-Verlag, New York, 1988, pp. 18-20) to determine the appropriate dosage to administer. An RGD sequence of the invention may be conveniently administered in unit dosage form, and may be prepared by any of the method well known in the pharmaceutical art, for example, as described in Remington's Pharmaceutical Sciences (Mack Pub. Co., Easton, PA 1990).

Physiologically acceptable and pharmaceutically acceptable excipients and carriers are well known to those of skill in the art. By "physiologically or pharmaceutically acceptable carrier" as used herein is meant any physiologically compatible substantially non-toxic carrier for administration in which an RGD sequence of the invention is bioavailable and does not cause excessive side effects (e.g., excessive bleeding) when used. For example, the RGD molecule of the invention can be dissolved in a liquid, dispersed, or emulsified in a medium in a conventional manner to form a liquid preparation or is mixed with a semi-solid (gel) or solid carrier to form a paste, ointment, cream, lotion or the like.

Suitable carriers include water, petroleum jelly (vaseline), petrolatum, mineral oil, vegetable oil, animal oil, organic and inorganic waxes, such as microcrystalline, paraffin and ozocerite wax, natural polymers, such as xanthanes, gelatin, cellulose, or gum arabic, synthetic polymers, such as discussed below, alcohols, polyols, water and the like. A water miscible carrier composition that is substantially miscible in water can be used. Such water miscible carrier composition can include those made with one or more ingredients set forth above but can also include sustained or delayed release carrier, including water containing, water dispersible or water soluble compositions, such as liposomes, microsponges, microspheres or microcapsules, aqueous base ointments, water-in-oil or oil-in-water emulsions or gels.

The carrier can comprise a sustained release or delayed release carrier. The carrier is any material capable of sustained or delayed release of the RGD sequence specifically directed against an αᵥβ₃ molecule to provide a more efficient administration resulting in one or more less frequent and/or decreased dosage of the RGD sequence, ease of handling, and extended or delayed effects. The RGD sequence is released from the carrier_when exposed to the environment of the area for treatment or diagnosis or by diffusing or by release dependent on the degree of loading of the sequence to the carrier in order to obtain release of the RGD peptide of the invention. Non-limiting examples of such carriers include liposomes, microsponges, microspheres, gene-activated matrices, as described above, or microcapsules of natural and synthetic polymers and the like. Examples of suitable carriers for sustained or delayed release in a moist environment include gelatin, gum arabic, xanthane polymers; by degree of loading include lignin polymers and the like; by oily, fatty or waxy environment include thermoplastic or flexible thermoset resin or elastomer including thermoplastic resins such as polyvinyl halides, polyvinyl esters, polyvinylidene halides and halogenated polyolefins, elastomers such as brasiliensis, polydienes, and halogenated natural and synthetic rubbers, and flexible thermoset resins such as polyurethanes, epoxy resins and the like. The sustained or delayed release carrier can be a liposome, microsponge, microsphere or gel. A pH balanced buffered aqueous solution for injection can be used. However, the preferred carrier will vary with the mode of administration. The compositions for administration usually contain from about 0.0001% to about 90% by weight of the RGD sequence compared to the total weight of the composition.

### SCREENING FOR αᵥβ₃ BINDING COMPOUNDS

Also included are method_of identifying compounds that inhibit αᵥβ₃ receptor-mediated binding of a cell having a cell proliferative disorder. For example, in a method for identifying a compound which inhibit αᵥβ₃ receptor-mediated binding of a glioma or prostate cell, the method includes incubating a test compound or agent and αᵥβ₃ under conditions sufficient to allow the compound and αᵥβ₃ to interact and detecting an effect of the compound or agent on αᵥβ₃ receptor-mediated binding. Conditions will vary according to a number of factors well recognized in the art, including whether the assay is performed in solution or in solid phase, *in vitro* or in intact cells, or *in vivo*, temperature, concentration, hydrophobicity and hydrophilicity of the molecules. For example, hydrophobic molecules such as long chain fatty acid may require buffers, including dimethylsulfoxide (DMSO). For interaction, such compounds or agents can thus be any number of molecules including polypeptides, peptidomimetics, carbohydrates, fatty acids, and/or steroids. The test compounds/agents, RGD molecules or αᵥβ₃ molecules may be labeled. For example, the αᵥβ₃ or the test compound may be chemically labeled with a fluorescence compound, a radioactive element or a metal chelating agent. Detection involves measuring the binding or an effect of binding of the compound to αᵥβ₃. In solution, in solid phase or in intact cells, bound and unbound compound can be separated by washing or fractionation or by any number of means including chromatography, gel electrophoresis and other means well known to those skilled in the ark.

In another embodiment, the invention provides methods_for identifying a glioma cell proliferative disorder that is susceptible to treatment with an RGD sequence. In one embodiment, the method includes contacting a cell such as a cell expressing αᵥβ₃ (e.g., a glioma or a prostate cell) with an RGD sequence under conditions which allow interaction between an RGD sequence and αᵥβ₃. Cells susceptible to RGD treatment are detected by measuring binding of the αᵥβ₃ cell to RGD compared to a non-RGD sequence. In another embodiment, cells susceptible to RGD treatment are detected by measuring binding of RGD to an intact (i.e., living) cell. In other embodiments, susceptible cells are detected by an increase in cell death or caspase activity.

The following specific examples are intended to further illustrate, but not limit, the present invention. Equivalents of the present invention will be easily identified by one skilled in the art.

### EXAMPLES

### Cell Cultures

Human glioblastoma, U-87MG, and U-373MG cells were obtained from the American Type Culture Collection, Rockville, MD and grown in Dulbecco's modified Eagle's medium supplemented with 10% fetal calf serum (Hyclone Laboratories Inc. Logan, UT), sodium pyruvate (55 mg/L), sodium bicarbonate (1.2 g/L), HEPES (1.2 g/L) and gentamicin (50 µg/ml). U-251MG cells were provided by Dr. G.Y. Gillespie, University of Alabama at Birmingham, Alabama, and were grown in the above media.

### Reagents

Peptides, cyclo(Arg-Gly-Asp-D-Phe-Val) or c-RGD represented by the formula below, and H-Arg-Gly-Asp-OH or RGD were purchased from BIOMOL Research Laboratories Inc., Plymouth Meeting, PA and Calbiochem, San Diego, CA, respectively.

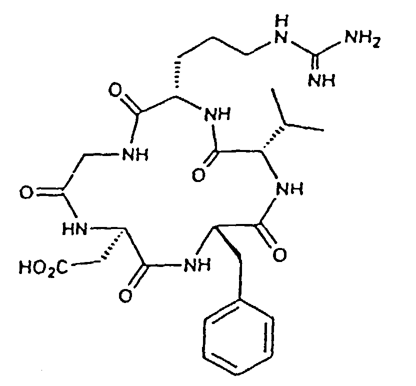

Stock solutions were made in sterile phosphate buffered saline (PBS), pH 7.2 (GIBCO BRL, Grand Island, NY). Fluorescein-conjugated anti-mouse IgG+IgM was obtained from Chemicon International, Inc., Temecula, CA. Monoclonal antibody to human integrin αᵥβ₃ was also purchased from Chemicon International, Inc. Specific inhibitors of caspase-3 and caspase-9, DEVD-FMK and LEHD-FMK, respectively, were purchased from Bio Vision Research Products, Palo Alto, California.

### Cell Surface Immunofluorescence Assay

Indirect immunofluorescence was performed according to Chatterjee *et al*., J. Virol. 44:1003-1012 (1982) and Chatterjee *et al*., Biochem. Biophys. Res. Comm. 167:1139-1145 (1990). Briefly, monolayers_of human glioma cells were washed with PBS and collected in PBS containing 0.1% bovine albumin (Sigma, St. Louis, MO) (PBS+BSA). The cells were centrifuged, washed in PBS+BSA buffer and treated with anti-αᵥβ₃ antibody (1:50 dilution) for 1 hour at 4° C with occasional shaking. Cells were then washed thoroughly with PBS+BSA buffer to remove unbound antibody_and fluorescein-conjugated anti-mouse IgG+IgM (1:50) was added. After incubation for one hour at 4 ° C, the cells were washed with PBS to remove unbound fluorescein-conjugated antibody before being mounted on glass microscope slides. Cell surface antigens were observed by a Nikon Eclipse E600 fluorescence microscope.

### Expression of αᵥβ₃ Integrin on the Surface of Human Glioma Cells

The indirect immunofluorescence assays demonstrated the expression of αᵥβ₃ integrin on the surface of U-87MG, U-373MG cells. The surface of U-251MG cells do not appear to have αᵥβ₃ integrin.

### Effect of cRGD and RGD Peptides on the Growth of Human Glioma Cells

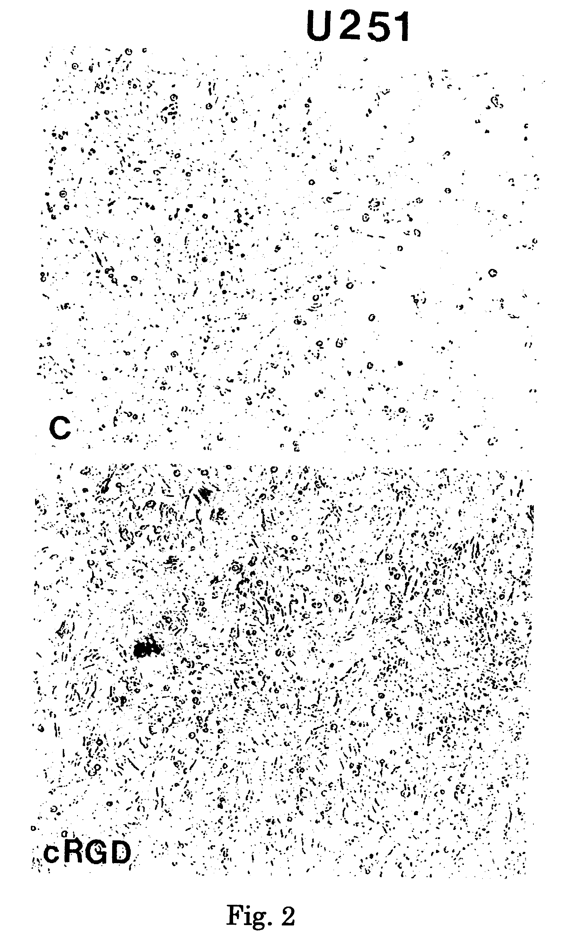

Semi-confluent monolayers of U-87MG, U-251MG and U-373MG cells were treated with different concentrations of cRGD for 18 hours at 37° C incubator with 5% CO₂. One set of cells served as untreated control. The cells were monitored under microscope for morphological changes. Most of the cRGD (10 µg/ml)-treated U-87MG (Figure 2; C=control) and U-373MG cells were rounded up. In contrast cRGD (10 µ g/ml)-treated U-251MG cells displayed no significant morphological changes in parallel experiment (Figure 2; C=control). The number of viable cells (both treated and untreated) were then counted by trypan blue dye exclusion method using a hemacytometer (Hausser Scientific, Horsham, PA) and percent inhibition was calculated (Table 1). This study_demonstrated that cyclic RGD peptides can significantly reduce the growth of αᵥβ₃ integrin-expressing human glioma cells *in vitro*.

### Effect of cRGD and RGD Peptides on the U-87MG-Induced Spheroid Formation

U-87MG cells can be grown either as a monolayer or as multicellular three-dimensional spheroids (500-1,000 µm) in liquid culture. Since spheroids closely resemble the architecture of a tumor *in vivo*, the cultures were contacted with cRGD or RGD to determine if treatment had any effect on spheroid formation. U-87MG cells (5 ×10⁵-1×10⁶) were mixed with either cRGD (100µg) or RGD (100µg) peptide or PBS (as untreated control) and incubated at 4° C for 30 minutes. Treated and untreated cells were then seeded onto 24-well plate and incubated further at 37° C. Forty eight hours later; the treated cells were treated again with respective peptides and incubated for 72 hours.

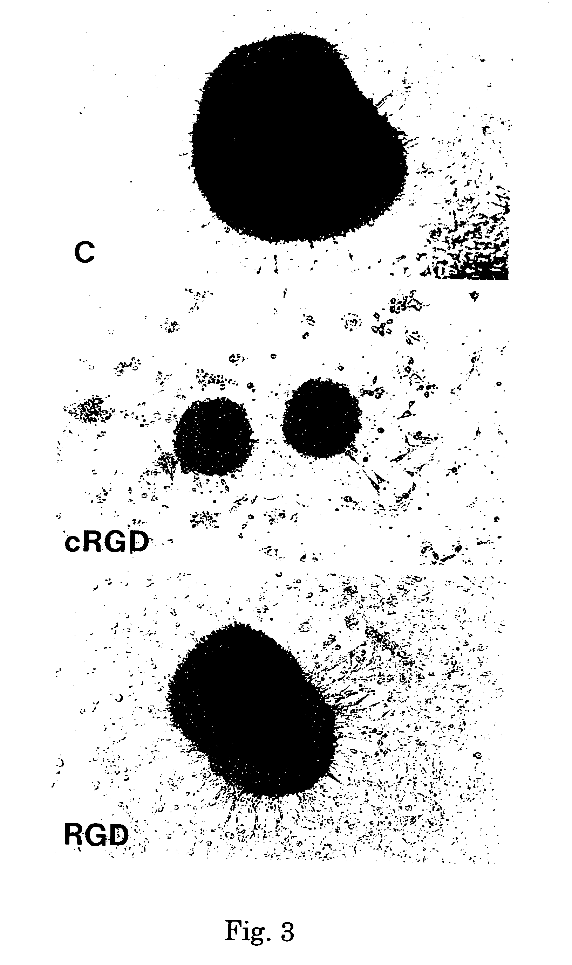

The result of this study shows that cRGD significantly reduced the size of the spheroids (by at least about 70%) when compared to that of untreated control (Figure 3; C=control). The untreated cell cultures produced large spheroids with dark central core and the surrounding cells were firmly attached and spreading (Figure 3). In contrast, the cRGD-treated culture had much smaller, poorly attached spheroids (or loose aggregates) and the surrounding cells were rounded off (Figure 3). Linear RGD at these concentration did not appear to effect spheroid formation when compared to the cRGD-treated U-87MG cells (Figure 3.) This is likely due, in part to a decreased affinity for αᵥβ₃ in comparison to cRGD. Thus, increasing the concentration of the linear RGD in this assay would likely to produce effects on spheroids similar to those observed for cRGD. The study demonstrated that cRGD inhibited the proliferation of human glioma cells.

In a second study, U-87MG cells were allowed to grow (in a 24-well plate) for 24 hours before the addition of cyclic (50 µg) or linear (50 µg) peptide. One set of cells, with PBS alone, served as untreated control. Forty eight hours later, the cells were treated again with respective cRGD (50 µg) or RGD (50 µg) peptides and incubated for an additional 72 hours. The result of this study demonstrated that cRGD had a significant inhibitory effect on the spheroid formation induced by U-87MG cells.

Thus, these studies clearly indicate that cyclic RGD can significantly block cell-to-cell adhesion, as well as proliferation of human glioma cells.

Moreover, these studies demonstrate the direct killing of human glioma cells by RGD peptides. This effect appeared to be specific for αᵥβ₃ integrin-expressing cells, like U-87MG or 373MG cells, but not for U-251MG cells, which do not appear to express αᵥβ₃ integrin on their membranes. It is possible that other glioma cells not expressing αᵥβ₃ may be susceptible to killing by RGD-containing molecules. For example, RGD may bind to other cell surface polypeptides on non-αᵥβ₃ expressing cells. Thus, these studies showing the effect of cRGD on glioma cells do not rule out the possibility of binding of cRGD to other glioma cells that do not express αᵥβ₃ integrin.

Treatment of U-87MG cRGD also significantly reduced the size of the spheroids in liquid culture. This assay is particularly important since a three dimensional spheroid closely resembles the architecture of a tumor *in vivo*. Thus, this type of assay likely predicts the outcome of *in vivo* studies more accurately.

cRGD appears to directly kill human glioma cells by inhibiting the cell-to-cell adhesion or cell proliferation *in vitro* which then leads_to cell detachment and death. This phenomenon appears different than blocking angiogenesis by antagonists, such as monoclonal antibody to integrin or cyclic RGD *in vivo* (see, Brooks, P. C., Montgomery; A. M. P., Rosenfeld, M., Reisfeld, R.A., Hu, T. *et al*. (1994) Cell 79:1157-1164; Brooks, P. C., Clark, R.A.F., Cheresh, D.A. (1994) Science 264:569-571).

### Effect of Caspase-3 and Caspase-9 Inhibitors on the Cell Death Induced by cRGDfV

To determine whether the observed killing of glioma cells was due to apoptosis, we used inhibitors of caspase-3 (DEVD-FMK) and caspase-9 (LEHD-FMK), two of the cysteine aspartyl proteases, known to be involved in apoptosis (see Salvesen GS, Dixit VM (1997) Cell 91:443-446; Cohen GM (1997) Biochem J326:1-16; Janicke RU, Sprengart ML, Wati MR, Porter AG (1998) J Biol Chem 273:9357-9360; Cardone MH, Roy N, Stennicke, HR, Salvesen GS, Franke TF, Stanbridge E, Frisch S, Reed JC (1998) Science 282:1318-1321; Sakahira H, Enari M, Nagata S (1998) Nature 391:96-99). Caspase-3 is required for morphological changes and cleavage of poly(ADP-ribose) polymerase (PARP), a cellular enzyme implicated in DNA repair mechanism. Caspase-9 is involved in cytochrome c-induced apoptosis by activating procaspase-3 which finally leads to apoptosis. Briefly, U-87MG and U-251MG cells were harvested from monolayer culture and were treated with DEVD-FMK (10 µM) or LEHD-FMK (10 µM) for 90 minutes at 37°C. One set of cells without any inhibitor served as control. Cells were then washed and incubated in the presence or absence of cRGDfV (20 µg/ml) at 37°C, 5% CO₂ for 18 hours. The number of viable cells, both in control and treated groups were counted and percent survival was determined.

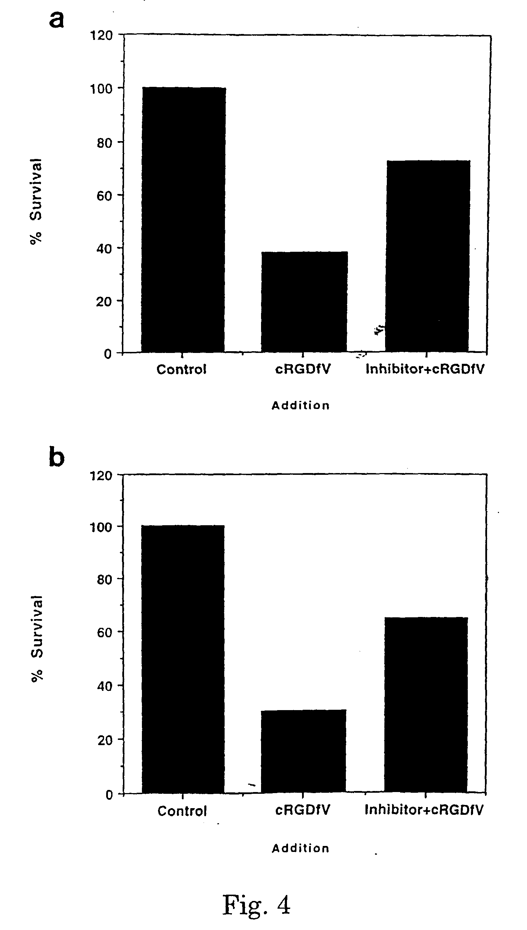

The inability of cRGDfV to reduce the number of viable U-87MG cell treated with these inhibitors (FIGS 4A and 4B) suggested that the induction of apoptosis of U-87MG cells was inhibited. The cell death we observed after cRGDfV treatment appeared to be due to apoptosis. In contrast, there is no significant effect of these inhibitors on the growth of U-251MG cells. These inhibitors, however, did not completely prevent killing of U-87MG cells, suggesting that other caspases might also be involved in cRGD-induced apoptosis of human glioma cells.

**TABLE 1**

| Cells | cRGDfY | | RGD | |
|---|---|---|---|---|
| | ug/ml | % inhibition | ug/ml | % inhibition |
| U-87MG | 0 | 0 | 0 | 0 |
| | 10 | 52.9 | 10 | 0 |
| | 20 | 70.6 | 20 | 3.5 |
| U-373MG | 0 | 0 | 0 | 0 |
| | 10 | 46.7 | 10 | 6.7 |
| | 20 | 80.0 | 20 | 0 |
| U-251MG | 0 | 0 | 0 | 0 |
| | 10 | 0 | 10 | 0 |
| | 20 | 5.9 | 20 | 13 |

Sequence Listing

<110> JCR Pharmaceuticals Co., Ltd.

<120> Modulation of Cell Proliferative Disorders by Cyclic RGD

<130> GP24

<150> US 60/108,387
<151> 1998-11-12

<160> 2

<210> 1
<211> 8
<212> PRT
<213> Artificial Sequence

<220>
<223> Designed peptide to include sequence Arg-Gly-Asp for binding to α v β 3 integrin.

<400> 1
Gly Arg Gly Asp Glu Pro Asp Gly
1               5

<210> 2
<211> 6
<212> PRT
<213> Artificial Sequence

<220>
<223> Designed peptide to include sequence Arg-Gly-Asp for binding to α v β 3 integrin.

The C-terminus is protected as an amide.

<400> 2

Arg-Gly-Asp-Asn-Ile-Glu

1 5

## Claims

1. A pharmaceutical composition for the treatment of an αᵥβ₃ receptor-mediated glioma or prostate cell proliferative disorder in the subject, wherein the composition contains as a pharmacologically active principle a cyclic RGD (cRGD) sequence.

2. The pharmaceutical composition of claim 1, wherein the cRGD sequence comprises a peptide sequence comprising Xₘ-Arg-Gly-Asp-Zₙ, wherein either of X and Z is natural or synthetic amino acid and either of m and n is an integer of 0 to 50.

3. The pharmaceutical composition of claim 2, wherein the cRGD sequence is the compound represented by the following formula.

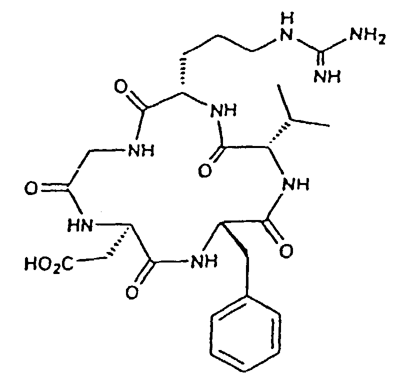

4. A pharmaceutical composition for the inhibition of occurrence of an αᵥβ₃₋receptor-mediated glioma prostate cell proliferative disorder in the subject, wherein the composition contains as a pharmacologically active principle a cyclic RGD (cRGD) sequence.

5. The pharmaceutical composition of claim 4, wherein the cRGD sequence comprises a peptide sequence comprising Xₘ-Arg-Gly-Asp-Zₙ, wherein either of X and Z is natural or synthetic amino acid and either of m and n is an integer of 0 to 50.

6. The pharmaceutical composition of claim 5, wherein the cRGD sequence is the compound represented by the following formula.

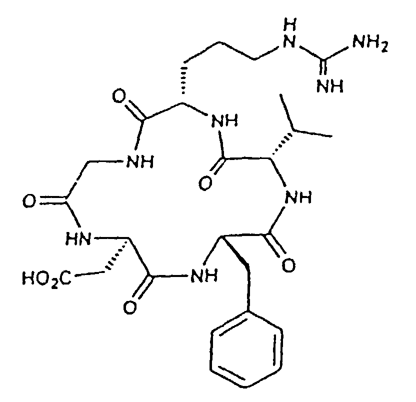

7. Use of a cyclic RGD (cRGD) sequence as a pharmacologically active principle for the manufacture of a composition for the treatment of an αᵥβ₃ receptor-mediated glioma or prostate cell proliferative disorder in the subject.

8. The use of claim 7, wherein the cRGD sequence comprises a peptide sequence comprising Xₘ-Arg-Gly-Asp-Zₙ, wherein either of X and Z is natural or synthetic amino acid and either of m and n is an integer of 0 to 50.

9. The pharmaceutical composition of claim 8, wherein the cRGD sequence is the compound represented by the following formula.

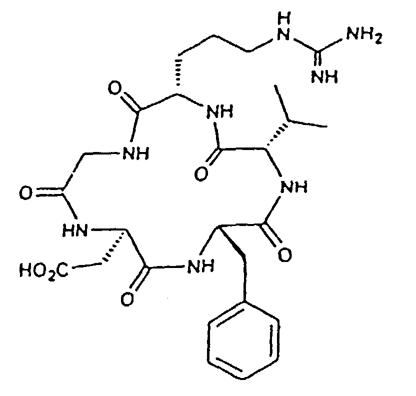

10. Use of a cyclic RGD (cRGD) sequence as a pharmacologically active principle for the manufacture of a composition for the inhibition of occurrence of an αᵥ β₃ receptor-mediated glioma or prostate cell proliferative disorder in the subject.

11. The use of claim 10, wherein the cRGD sequence comprises a peptide sequence comprising Xₘ-Arg-Gly-Asp-Zₙ, wherein either of X and Z is natural or synthetic amino acid and either of m and n is an integer of 0 to 50.

12. The use of claim 11, wherein the cRGD sequence is the compound represented by the following formula.

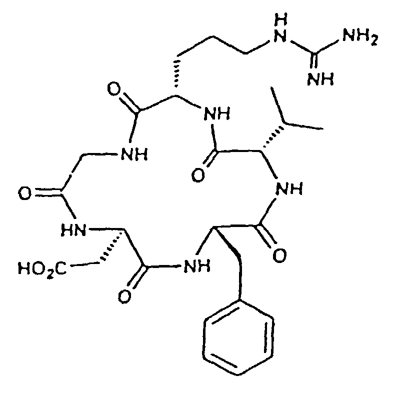

13. A method for identifying a compound which modulates an αᵥβ₃ receptor-mediated glioma or prostate cell proliferative disorder, comprising contacting αᵥβ₃ glioma or prostate cell with a candidate compound and detecting binding of the compound to αᵥβ₃

14. The method of claim 13, wherein the compound comprises an RGD sequence.

15. The pharmaceutical composition of one of claims 1 to 6 further containing at least one chemotherapeutic agent.

16. The pharmaceutical composition of claim 15, wherein the chemotherapeutic agent is selected from the group consisting of bleomycin, neocarcinostatin, suramin, doxorubicin, taxol, mitomycin C and cisplatin.

17. A method for identifying an agent which inhibits glioma or prostate cell proliferation comprising contacting a sample containing an αᵥβ₃ receptor with a candidate compound and detecting an effect on αᵥβ₃ receptor-mediated binding.

18. The method of claim 17, wherein the compound is selected from the group consisting of a peptide, a peptidomimetic and an antibody.
